# FASCICULE DE BREVET EUROPEEN

(11) **EP 0 891 192 B1**
(45) Date de publication et mention de la délivrance du brevet: **16.04.2003**
(21) Numéro de dépôt: 97913354.3
(22) Date de dépôt: 02.12.1997
(51) Int. Cl.: A61L 2/08

(54) **INSTALLATION DE STERILISATION DE PRODUITS PAR IRRADIATION**
ANLAGE ZUM STERILISIEREN VON PRODUKTEN DURCH BESTRAHLUNG
INSTALLATION FOR STERILISING PRODUCTS BY RADIATION

(30) Priorité: 03.12.1996 IT TO960980
(43) Date de publication de la demande: 20.01.1999
(73) Titulaire: GAMBRO HOSPAL (Schweiz) AG, 4001 Basel (CH)
(72) Inventeur: CASTELLANI, Antonio, I-41100 Modena (IT)
(74) Mandataire: Lejeune, Daniel
(86) Numéro de dépôt international: IB9701502
(87) Numéro de publication internationale: WO98024484

(56) Documents cités:
- WO-A-95/23616
- DE-U- 8 913 860
- GB-A- 764 337
- GB-A- 1 525 484
- US-A- 4 983 849

## Description

La présente invention concerne une installation de stérilisation de produits par irradiation.

Il est connu que les installations de stérilisation de produits par irradiation, par exemple, au moyen d'électrons accélérés à haute énergie cinétique ("bêta") comprennent:
- un poste d'irradiation isolé de l'environnement, à l'intérieur duquel est disposé un accélérateur de particules;
- un convoyeur d'entrée qui achemine le produit à stériliser dans le poste d'irradiation pour le placer sous de l'accélérateur, et
- un convoyeur de sortie qui transporte le produit irradié à l'extérieur du poste d'irradiation.

Etant donné que la permanence des radiations de type ionisant provoque d'importants dommages dans l'environnement, tels qu'une dégradation des matériaux, des phénomènes d'électrolyse, la formation d'ozone, dans la zone d'irradiation et au dessous du trajet d'avancement des produits à irradier, il est prévu un piège d'absorption (dispositif d'arrêt des faisceaux) pour le faisceau β formé généralement par une cible refroidie qui intercepte le faisceau et dissipe l'énergie résiduelle transformée en chaleur. En outre, pour l'évacuation de l'ozone, il est prévu des circuits d'aération forcée pour évacuer le plus rapidement possible l'ozone formée.

Toutefois, étant donné le trajet relativement important des rayons ionisants dans les pièges d'absorption connus, entre le point d'émission et le dispositif d'arrêt des faisceaux, on ne peut pas réduire le trajet dans l'air du faisceau β. D'autre part, la quantité d'ozone produite est proportionnelle au trajet effectué dans l'air. On sait que l'ozone est un gaz nocif pour les personnes et dangereux pour les matériaux.

En outre, étant donné les caractéristiques de l'interaction entre les électrons accélérés et la matière, il n'est généralement pas possible d'irradier le produit de manière adéquate, à la fois en profondeur et sur les bords, sans créer d'importantes différences d'exposition et provoquer une dégradation des matériaux dans les zones surexposées. Dans le cas de produits délicats (tels que les conduites ou lignes à sang en PVC pour appareils de dialyse) il est nécessaire d'exposer le produit à deux reprises à l'irradiation, sous deux faces différentes, pour assurer la stérilisation des zones insuffisamment traitées lors d'un premier passage sous la source d'irradiation. Cette solution coûteuse, qu'il n'est pas toujours possible de mettre en pratique, ne permet pas toujours d'éviter la détérioration des zones traitées à deux reprises étant donné que, si elle permet de résoudre le problème de la pénétration de la radiation dans le produit, elle provoque une aggravation sensible du manque d'uniformité de l'exposition (c'est-à-dire une élévation du surdosage). Dans tous les cas, cette solution ne permet pas d'améliorer notablement l'efficacité de l'irradiation sur les bords des produits.

GB-A-1525484 décrit une installation de stérilisation de récipients destinés à être remplis et scellés, comprenant un accélérateur d'électrons orienté sur la trajectoire d'un tapis transportant les récipients à stériliser. Une plaque réflectrice en plomb est disposée en-dessous desdits récipients afin de renvoyer le faisceau d'électrons vers la partie inférieure des récipients.

Le but de la présente invention consiste à réaliser une installation de stérilisation permettant d'homogénéiser la dose d'irradiation reçue par les différentes zones du produit, d'assurer en même temps une efficacité supérieure du procédé et de réduire l'influence nocive de l'irradiation sur l'environnement.

Selon la présente invention, on réalise une installation de stérilisation de produits par irradiation comprenant un poste d'irradiation doté d'un accélérateur de particules et d'un piège d'absorption comprenant un corps réflecteur muni de moyens de refroidissement.

D'autres avantages et caractéristiques de l'invention apparaîtront à la lecture de la description qui suit. On se reportera aux dessins annexés sur lesquels:
La figure 1 est une vue schématique en perspective de la présente installation,
Les figures 2 et 3 sont des courbes représentant la pénétration de la radiation dans un produit obtenue, respectivement, dans une installation classique et dans une installation selon l'invention, et
la figure 4 est une courbe représentant le profil transversal de l'irradiation produite une installation classique.

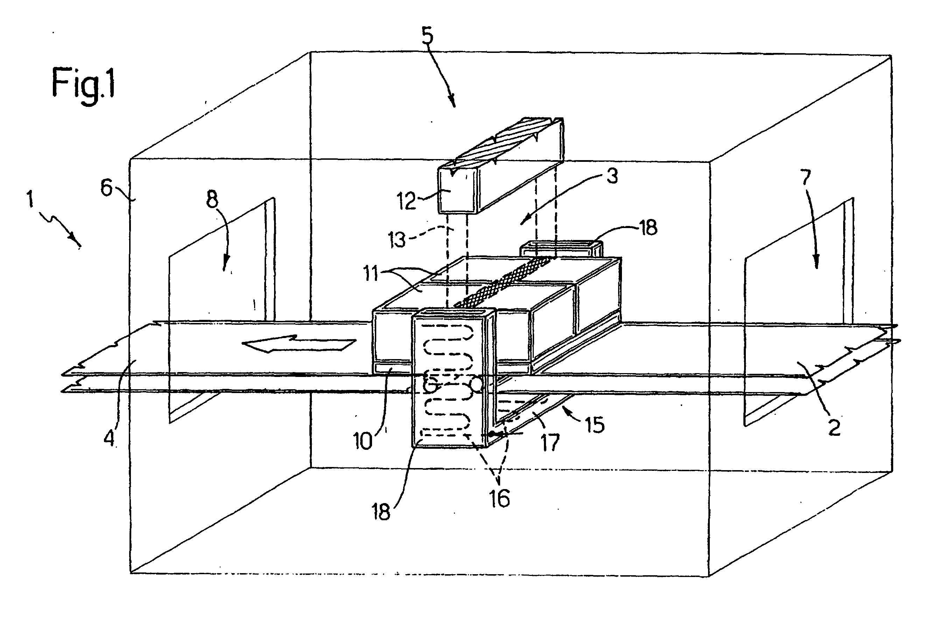

En se référant à la figure 1, l'installation de stérilisation est désignée, dans son ensemble, par le chiffre de référence 1. L'installation 1 comprend un convoyeur d'entrée 2, une zone d'irradiation 3 et un convoyeur de sortie 4. La zone d'irradiation 3 est enfermée dans une chambre de traitement 5, isolée par rapport à l'environnement extérieur par des parois 6, illustrées seulement schématiquement et est dotée d'une installation de ventilation forcée (non représentée). Les convoyeurs d'entrée et de sortie 2, 3, pénètrent et sortent de la chambre de traitement 5 par des ouvertures correspondantes 7, 8. Comme représenté schématiquement, les convoyeurs d'entrée 2 et de sortie 4 ne s'étendent pas sous la zone d'irradiation 3, pour éviter les dommages causés par une irradiation directe.

Les convoyeurs 2, 4 transportent un plateau 10 supportant le produit à traiter 11 (sur le dessin, deux boîtes disposées côte à côte contenant, par exemple, des lignes à sang pour appareils de dialyse).

Un accélérateur d'électrons 12 comprenant une tête de balayage est disposé au-dessus de la zone d'irradiation 3, de manière à produire un faisceau 13 d'électrons accélérés irradiant une portion de produit, comme représenté schématiquement sur la figure 1.

Comme représenté sur la figure, un piège d'absorption 15 est disposé dans la chambre de traitement 5 en regard de la zone d'irradiation 3, légèrement au dessous du niveau des convoyeurs 2, 4. Le piège 15 est constitué par un corps en matériau métallique à haut pouvoir de rétrodiffusion, par exemple, en plomb ou en fer, contenant à l'intérieur un serpentin 16 (représenté schématiquement sur la figure) constitué, de préférence, par un tuyau de cuivre. Le piège 15 est en forme de U et comprend:
- une partie inférieure 17 parallèle au plan des convoyeurs 2, 4 et perpendiculaire au faisceau 13, et
- deux parois latérales 18, dirigées vers le haut respectivement à partir de chaque extrémité de la partie inférieure 17, latéralement par rapport au trajet du plateau 10.

Les dimensions du piège 15 sont telles qu'elles permettent d'intercepter la totalité du faisceau d'électrons 13 afin de protéger les'structures et les équipements présents dans la chambre de traitement 5. Le serpentin 16 est traversé par un liquide de refroidissement pour dissiper la puissance du faisceau 13. En particulier, il est dimensionné de manière à pouvoir dissiper la totalité de la puissance du faisceau, de façon que l'accélérateur 9 puisse être mis en fonctionnement, même en l'absence de produit à irradier.

Le piège réflecteur 15 a pour fonction de réfléchir vers le haut (selon le phénomène de rétrodiffusion) les électrons accélérés du faisceau 13 qui vont au-delà du produit à traiter 11, ce qui permet de compenser partiellement la réduction de la dose absorbée par le produit à mesure que le faisceau pénètre dans l'épaisseur du produit.

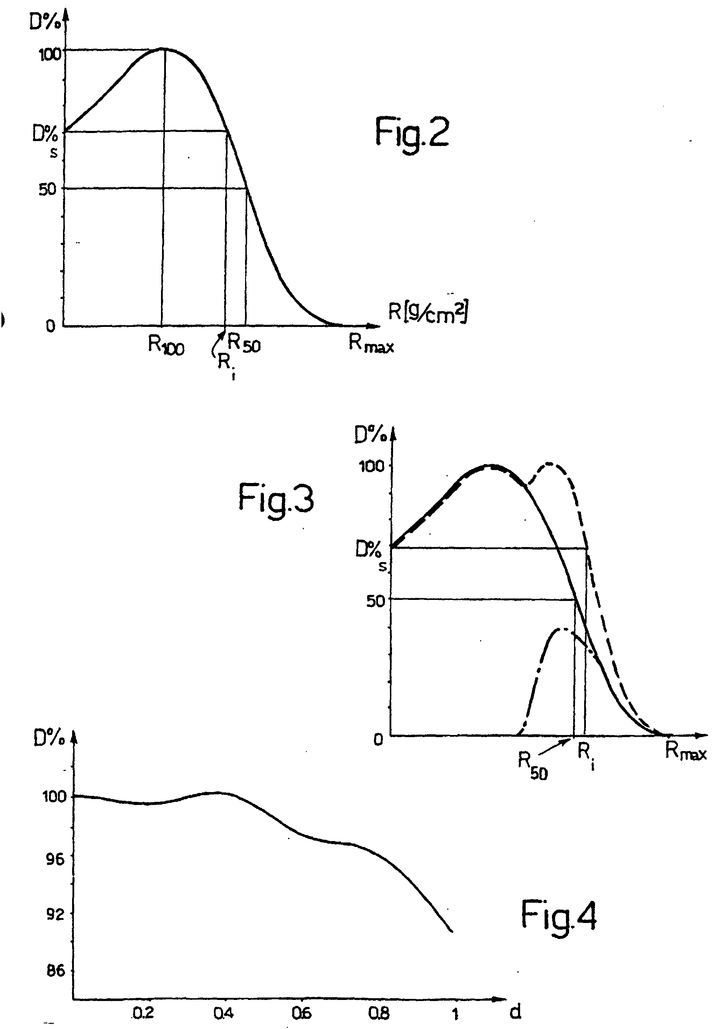

A cet égard, on se réfère à la figure 2 qui représente l'allure caractéristique classique de la dose absorbée en fonction de la profondeur d'un produit caractérisé par une densité uniforme. Sur la figure 2, on a porté en abscisse la profondeur R du produit, exprimée par l'épaisseur efficace (g/cm2, obtenue en multipliant la densité du matériau par l'épaisseur traversée) et on a porté en ordonnée le pourcentage de dose absorbée D%. Comme on peut le voir, la courbe D% présente un premier tronçon, à partir de la surface du produit traité jusqu'à une profondeur indiquée par R₁₀₀, où la valeur superficielle D%_{S} augmente jusqu'à une valeur maximale égale à 100%, et un second tronçon, à partir de R₁₀₀ jusqu'à une valeur de profondeur maximale susceptible d'être atteinte Rₘₐₓ, où la dose absorbée diminue rapidement et peut être insuffisante pour le traitement de stérilisation requis (en général, l'épaisseur admissible est celle à laquelle la dose est supérieure à D%_{S} ou lorsque R est compris entre 0 et un point indiqué par R1 sur la figure 2). En outre, si l'épaisseur totale du produit à traiter est inférieure à la profondeur maximale admissible Rₘₐₓ, une partie du faisceau produit traverse le produit sans transférer toute son énergie.

En revanche, dans l'installation selon l'invention qui comprend le piège réflecteur 15, cette fraction résiduelle du faisceau n'est pas perdue mais est utilisée sous forme d'une irradiation secondaire par "rétrodiffusion". La courbe de l'irradiation secondaire susceptible d'être produite au moyen du piège réflecteur 15 est représentée en traits mixtes sur la figure 3 où la profondeur est mesurée dans le système de référence de l'irradiation primaire, de sorte que ce système est représenté par un pic correspondant de la partie décroissante de la courbe primaire (partie inférieure du matériau à traiter).

L'irradiation secondaire s'ajoute à l'irradiation primaire, ce qui donne la courbe illustrée par la ligne en traits tirés sur la figure 3, qui comprend une première partie identique à celle de la courbe d'irradiation primaire, et une seconde partie dans laquelle le pourcentage de dose absorbée reste à une valeur supérieure à la valeur obtenue sur la surface D%_{S}, pour un intervalle de plus grande profondeur. II est ainsi possible d'obtenir une irradiation adéquate, même dans le cas d'un produit d'épaisseur supérieure, sans qu'il soit nécessaire d'effectuer un double passage et de risquer de détériorer le produit et sans avoir à modifier les paramètres du procédé, ce qui permet ainsi d'assurer une meilleur efficacité du traitement par irradiation.

La présence des parois latérales 18 rend, en outre, possible les effets de bord de l'irradiation. De fait, l'interaction entre un faisceau de radiation et les particules du produit à traiter crée des phénomènes locaux de rétrodiffusion qui contribuent au traitement. Toutefois, dans les zones périphériques du produit, en raison de la discontinuité constituée par l'interface produit-air, il se produit une faible contribution locale qui détermine une baisse de rendement du traitement par rapport aux zones centrales. Ce phénomène est mis en évidence sur la courbe de la figure 4 qui représente l'allure de la courbe du pourcentage de la dose absorbée D% en fonction de la distance d entre chaque point et le centre du produit (en prenant la distance centre-bord égale à 1). Comme on peut le voir, la dose absorbée est assez constante et avoisine 100% dans la zone centrale du produit, pour baisser diminuer à l'approche de la périphérie.

Grâce à la présence des parois latérales 18, il se produit une rétrodiffusion de la radiation déviée latéralement vers les zones périphériques du produit, et cette rétrodiffusion assure une compensation de la baisse de l'efficacité du traitement sur les bords, analogue à ce qui se produit dans la zone inférieure du produit.

La disposition du piège réflecteur 15, immédiatement au dessous du trajet d'avancement du produit 11 permet de réduire le parcours dans l'air du faisceau 13, ce qui limite la production d'ozone (nocive pour l'homme et agressive vis-à-vis des organes mécaniques présents dans la chambre de traitement 5). Le serpentin 16 permet de dissiper l'énergie des électrons accélérés, non réfléchis par le piège réflecteur 15.

La présence du piège réflecteur 15 permet enfin l'utilisation d'un plateau 10 en matériau métallique perforé qui contribue à la réflexion du faisceau vers le haut et limite l'échauffement excessif du plateau sous l'effet de l'irradiation (le plateau peut donc être manipulé après sa sortie de la chambre de traitement).

Grâce à la présence du piège réflecteur décrit, la présente invention est d'une efficacité élevée et garantit l'uniformité de stérilisation des produits. Elle permet de traiter de manière adéquate même des produits délicats tels que des lignes de sang et a un impact limité sur l'environnement.

La présente invention n'est pas limitée au mode de réalisation décrit et représenté et elle est susceptible de variantes.

## Revendications

1. Installation de stérilisation (1) de produits (11) par irradiation munie d'un poste d'irradiation (3) comportant un accélérateur de particules (12) et un piège d'adsorption (15) disposés de part et d'autre d'un trajet de transport des produits à stériliser (11), le piège d'adsorption (15) comprenant un corps réflecteur de particules, **caractérisée en ce que** le corps réflecteur de particules comprend des moyens de refroidissement (16).

2. Installation selon la revendication 1, **caractérisée en ce que** le corps réflecteur (15) est en matériau métallique, tel que le plomb ou le fer.

3. Installation selon la revendication 1 ou 2, **caractérisée en ce qu'**elle comprend un premier et un second convoyeurs (2, 4) disposés dans le prolongement l'un de l'autre et séparés par un interstice situé à l'aplomb de l'accélérateur de particules (12), les convoyeurs (2,4) définissant le trajet de transport des produits à stériliser (11) et le corps réflecteur (15) étant disposé immédiatement au dessous du trajet de transport, en vis-à-vis de l'accélérateur de particules (12).

4. Installation selon la revendication 3, **caractérisée en ce que** le corps réflecteur (15) a la forme d'un U comprenant une partie inférieure (17) et deux parois latérales (18) se dressant respectivement aux extrémités de la partie inférieure, la partie inférieure (17) étant disposée au dessous du trajet de transport sur toute la largeur d'irradiation, et les parois latérales (18) faisant saillie vers l'accélérateur de particules (12).

5. Installation selon l'une quelconque des revendications précédentes **caractérisée en ce que** les moyens de reroidissement du corps réflecteur (15) comprennent un serpentin (16) pour le passage d'un liquide de refroidissement.

6. Piège d'absorption (15) pour une installation de stérilisation par irradiation (1), **caractérisé en ce qu'**il comprend un corps réflecteur tel que défini dans l'une quelconque des revendications précédentes.

## Claims

1. Plant (1) for sterilizing products (11) by irradiation, the said plant being provided with an irradiation unit (3) comprising a particle accelerator (12) and an adsorption trap (15) that are placed one on each side of a path for transporting the products (11) to be sterilized, the adsorption trap (15) comprising a particle-reflecting body, **characterized in that** the particle-reflecting body includes cooling means (16).

2. Plant according to Claim 1, **characterized in that** the reflecting body (15) is made of a metallic material, such as lead or iron.

3. Plant according to Claim 1 or 2, **characterized in that** it includes a first and a second conveyor (2, 4) placed one in the extension of the other and separated by a gap located vertically in line with the particle accelerator (12), the conveyors (2, 4) defining the transport path of the products to be sterilized (11) and the reflecting body (15) being placed immediately beneath the transport path facing the particle accelerator (12).

4. Plant according to Claim 3, **characterized in that** the reflecting body (15) is in the form of a U, comprising a lower part (17) and two side walls (18) standing up at the respective ends of the lower part, the lower part (17) being placed beneath the transport path over the entire irradiation width and the side walls (18) projecting towards the particle accelerator (12).

5. Plant according to any one of the preceding claims, **characterized in that** the means for cooling the reflecting body (15) comprise a coil (16) for the circulation of a cooling liquid.

6. Absorption trap (15) for an irradiation sterilization plant (1), **characterized in that** it comprises a reflecting body as defined in any one of the preceding claims.

## Patentansprüche

1. Sterilisationsanlage (1) für Produkte (11) durch Bestrahlung, die mit einer Bestrahlungseinrichtung (3) versehen ist, die einen Teilchenbeschleuniger (12) und eine Absorptionsfalle (15) umfasst, die auf beiden Seiten einer Transportbahn für zu sterilisierende Produkte (11) angeordnet sind, wobei die Absorptionsfalle (15) einen Reflektorkörper für Teilchen umfasst, **dadurch gekennzeichnet, dass** der Reflektorkörper für Teilchen Kühlmittel (16) umfasst.

2. Anlage nach Anspruch 1, **dadurch gekennzeichnet, dass** der Reflektorkörper (15) aus metallischem Material besteht, wie beispielsweise Blei oder Eisen.

3. Anlage nach Anspruch 1 oder 2, **dadurch gekennzeichnet, dass** sie eine erste und eine zweite Fördereinrichtung (2, 4) umfasst, die in Verlängerung voneinander angeordnet und durch einen Zwischenraum getrennt sind, der senkrecht zum Teilchenbeschleuniger (12) liegt, wobei die Fördereinrichtungen (2, 4) die Transportbahn der zu sterilisierenden Produkte (11) definieren und der Reflektorkörper (15) unmittelbar unterhalb der Transportbahn gegenüber dem Teilchenbeschleuniger (12) angeordnet ist.

4. Anlage nach Anspruch 3, **dadurch gekennzeichnet, dass** der Reflektorkörper (15) eine U-Form mit einem unteren Teil (17) und zwei Seitenwänden (18) hat, die jeweils an den Enden des unteren Teils aufragen, wobei der untere Teil (17) unter der Transportbahn auf der ganzen Bestrahlungsbreite angeordnet ist und die Seitenwände (18) zum Teilchenbeschleuniger (12) hin ragen.

5. Anlage nach einem der vorhergehenden Ansprüche, **dadurch gekennzeichnet, dass** die Kühlmittel des Reflektorkörpers (15) eine Rohrschlange (16) für den Durchtritt einer Kühlflüssigkeit umfassen.

6. Absorptionsfalle (15) für eine Anlage zur Sterilisierung durch Strahlung (1), **dadurch gekennzeichnet, dass** sie einen Reflektorkörper umfasst, wie er in einem der vorhergehenden Ansprüche definiert ist.
